(19) Europäisches Patentamt
European Patent Office
Office européen des brevets

(11) **EP 1 850 743 B1**

(12) **EUROPEAN PATENT SPECIFICATION**

(45) Date of publication and mention
of the grant of the patent:
**05.12.2012 Bulletin 2012/49**

(51) Int Cl.:
***A61B 5/05*** *(2006.01)*    ***A61B 5/00*** *(2006.01)*

(21) Application number: **06704227.5**

(22) Date of filing: **30.01.2006**

(86) International application number:
**PCT/GB2006/000303**

(87) International publication number:
**WO 2006/085052 (17.08.2006 Gazette 2006/33)**

(54) **METHODS AND APPARATUS FOR MEASURING THE INTERNAL STRUCTURE OF AN OBJECT**

VERFAHREN UND GERÄT ZUR MESSUNG DER INNEREN STRUKTUR EINES OBJEKTS

PROCEDES ET APPAREILS PERMETTANT DE MESURER LA STRUCTURE INTERNE D'UN OBJET

(84) Designated Contracting States:
**AT BE BG CH CY CZ DE DK EE ES FI FR GB GR HU IE IS IT LI LT LU LV MC NL PL PT RO SE SI SK TR**

(30) Priority: **09.02.2005 GB 0502651**

(43) Date of publication of application:
**07.11.2007 Bulletin 2007/45**

(73) Proprietor: **Micrima Limited**
**Temple Quay**
**Bristol**
**BS1 6EG (GB)**

(72) Inventors:
• **CRADDOCK, Ian**
**Bristol BS9 3UG (GB)**
• **PREECE, Alan William**
**Bristol BS9 2BW (GB)**
• **NILAVALAN, Rajagopal**
**Dept. of Electronics&Comp.**
**Middlesex UB8 3PH (GB)**
• **LEENDERTZ, Jack Albert**
**Bristol BS7 9DZ (GB)**
• **BENJAMIN, Ralph**
**Kingsweston**
**Bristol BS11 0UE (GB)**
• **WILSON, Frederick John**
**9 Sprats Barn Crescent**
**Wiltshire SN4 7JP (GB)**

(74) Representative: **Ribeiro, James Michael et al**
**Withers & Rogers LLP**
**4 More London Riverside**
**London**
**SE1 2AU (GB)**

(56) References cited:
**WO-A-03/003907**    **WO-A-03/009753**
**US-A1- 2004 077 943**

• **HAGNESS S C ET AL: "Two-dimensional FDTD analysis of a pulsed microwave confocal system for breast cancer detection: fixed-focus and antenna-array sensors" IEEE TRANSACTIONS ON BIOMEDICAL ENGINEERING, IEEE SERVICE CENTER, PISCATAWAY, NJ, US, vol. 45, no. 12, December 1998 (1998-12), pages 1470-1479, XP002201090 ISSN: 0018-9294**

**Description**

The present invention relates to a method and apparatus for measuring the internal structure of an object, such as a human breast.

Breast cancer is the most common cancer in woman - in the UK, nearly 1 in 3 of all cancers in women occur in the breast, with a lifetime risk of 1 in 9 - see http://www.breastcancercare.org.uk/Breastcancer/Breastcancerfactsandstatistics. Among the currently available breast screening methods X-ray mammography is considered the most effective technique. See M. Brown, F. Houn, E. Sickles and L. Kessler, Screening mammography in community practice, Amer. J. Roentgen, vol. 165, pp. 1373-1377, Dec. 1995. However this technique suffers from relatively high false negative and positive detection rates, involves uncomfortable compression of the breast (see P. T. Huynh, A. M. Jarolimek and S .Daye, The false negative mammogram, Radiograph, vol.18, pp. 1137-1154, 1998) and is not well-suited to denser breasts (see E. Banks et al, Influence of personal characteristics of individual women on sensitivity and specificity of mammography in the Million Women Study: cohort study, British Medical Journal, vol. 329(7464):477, August 2004). The ionising nature of X-ray exposure is also a matter of concern.

Microwave radar-based detection of breast cancer is a non-ionising alternative that is being studied by a number of groups world-wide. See for example Xu Li and S. C. Hagness, A confocal microwave imaging algorithm for breast cancer detection, IEEE Microwave & Wireless Components Lett., vol.11, pp. 130-2, Mar. 2001; E. C. Fear and M. A. Stuchly, Microwave system for breast tumour detection, IEEE Microwave & Guided Wave Lett., vol. 9, pp 470-2, Nov. 1999; and P. M. Meaney, M. W. Fanning, D. Li, S. P. Poplack and K. D. Paulsen, Clinical prototype for active microwave imaging of the breast, IEEE Trans. on Microwave Theory and Tech., vol. 48, pp. 1841-1853, Nov. 2000. All such methods rely upon the difference in permittivity between malignant and normal breast tissue (between 2:1 and 10:1, depending on the density of normal tissue). Microwave attenuation in normal breast tissue is less than 4 dB/cm up to 10GHz (see S. C. Hagness, A. Taflove, and J. E. Bridges, Two-dimensional FDTD analysis of a pulsed microwave confocal system for breast cancer detection: fixed-focus and antenna-array sensors, IEEE Trans. on Biomed. Eng., vol. 45, pp. 1470-9, Dec. 1998) and this frequency range should permit sufficiently good spatial resolution after focusing.

A microwave radar technique employing a Real Aperture Synthetically Organised Radar detection method originally developed for land mine detection is described in R. Benjamin, I. J. Craddock, G. S. Hilton, S. Litobarski, E. McCutcheon, R. Nilavalan, G. N. Crisp, Microwave detection of buried mines using non-contact, synthetic near-field focusing. IEE Proceedings: Radar, Sonar & Navigation, vol. 148, pp. 233-40, Aug. 2001; and in R. Benjamin, Post-Reception Focusing in Remote Detection Systems, US patent US-A-5,920,285.

A problem with any imaging technique that transmits wave energy into the object is that reflections from the surface of the object can cause unwanted signal artifacts - this can be particularly serious when there is a surface skin of higher density than the medium inside the object. WO 03/009753 discloses a method of measuring the internal structure of an object having an antenna array wherein one antenna is transmitting and several antennas receive in multiple receive channels. The skin artifact is removed by a method by which the skin artifact at each antenna may be estimated as a filtered combination of the signal at all other antennas. The signals from each of the other antennas are provided to FIR filters, the outputs of which are summed and subtracted from the signal from the particular antenna after a delay. The inventions discussed below present various solutions for reducing such signal artifacts.

A first aspect of the invention provides a method of measuring the internal structure of an object according to claim 9.

The first aspect of the invention provides a processing method to remove surface reflection artifacts. High resolution is achieved by operating over a range of frequencies.

In a special case, step c) may be performed for only one subset. However, in general step c) will be performed a plurality of times, each instance relating to a different subset of output signals.

In a special case, the number of output signals in the subset may be equal to the total number of output signals generated by the receivers. However, in most cases the number of output signals in he subset is smaller than the total number of output signals generated by the receivers.

Only a single transmitter may be used, or a plurality of transmitters. The transmitters are typically microwave antennas or ultrasound transducers. In a preferred embodiment the antennas/transducers are energized sequentially so as to transmit a series of wave pulses onto the object, as described in US-A-5,920,285. Any antenna/transducer not acting as a transmitter, acts as a receiver (reception by the transmitting antenna could also be included, but this is not preferred). In this case, only one transmitter can be transmitting at any one time, and each pulse contains frequency components spanning a range of frequencies. However, alternatively each transmitter may transmit a sinusoidal signal whose frequency is varied over a range. In other embodiments, by offsetting swept-frequency signals, more than one transmitter can then be energised at the same time. Alternatively, a "code-division multiplexed" system may be employed, in which each transmitter transmits a unique encoded signal, enabling more than one transmitter to be energised at the same time.

In one embodiment, step ii) includes selecting one of the output signals in the subset as a calibration signal, for instance by selecting the signal which results in the smallest integral of the square difference between this signal and one other member of the subset of output signals. The calibration signal is then subtracted from the one other member in step iii). In general this process will be repeated for each member of the subset, resulting in a different calibration signal for each member of the subset. In another embodiment, step ii) includes calculating an average of the subset of output signals, which may be a weighted average. This average calibration signal is then subtracted from each member of the subset.

The first aspect of the invention requires relatively broadband signal processing. Therefore typically the calibration signal contains frequency components spanning a range having a width which is greater than 50% of the centre-frequency. In a microwave implementation of the imaging system this would imply typically a width greater than 1 GHz and most preferably greater than 4 GHz.

The first aspect of the invention also provides apparatus for measuring the internal structure of an object according to claim 15.

Preferably the apparatus includes

    a) a transmitter for transmitting wave energy onto the object;
    b) a receiver for detecting the effect of the object on the passage of the wave energy to generate an output; and
    c) a blocking member positioned between the transmitter and receiver, the blocking member being arranged so as to fully or partially block wave energy reflected from a surface of the object before it reaches the receiver.

The blocking member is positioned so as to partially or fully block reflected energy, and hence reduce reflected signal artifacts.

As well as reducing signal artifacts due to reflections from the surface of the object, if the blocking member is positioned in a direct line of sight between the transmitter and receiver, then artifacts due to direct coupling between the transmitter and receiver can also be reduced.

Typically the blocking member includes a screening material which does not allow waves to pass through. In the radar case the screening material will be a metal such as aluminium. Additionally, or as an alternative, the blocking member may include an attenuating material which absorbs waves. In a preferred case an attenuating material is provided as a coating on a substrate of screening material.

Typically the transmitter and receiver comprise an array of antennas, and a blocking member is positioned between each pair of adjacent antennas in the array.

The blocking member may be a perforated mesh, but preferably is in the form of a continuous screen.

The method may include the steps of:

    a) transmitting wave energy onto the object;
    b) detecting the effect of the object on the passage of the wave energy to generate an output;
    c) positioning a blocking member adjacent to, or in contact with, a surface of the object; and
    d) fully or partially blocking wave energy reflected from the surface of the object with the blocking member.

Preferably the apparatus includes

    a) a transmitter configured to transmit wave energy at a frequency f;
    b) an anti-reflective layer which, at the frequency f, has a thickness of a quarter-wavelength in the refractive index of the anti-reflective layer; and
    c) a receiver for detecting the effect of the object on the passage of the wave energy to generate an output.

The anti-reflective layer lies in the path between the transmitter and the receiver via the object, and is in contact or in very close proximity to the surface of the object. The anti-reflection layer is designed in order that, when a wave is incident upon it, the reflected wave is similar in amplitude, but opposite in phase, to the one from the surface of the object so as to result in destructive interference. This is accomplished by tailoring the thickness of the layer, by giving it a thickness of one quarter wavelength at the given refractive index and operating frequency *f*.

Typically the anti-reflective layer includes a resin-based material, which may be water-loaded and/or aluminium-loaded.

The anti-reflective layer may have a curved surface, for instance shaped to conform to the contour of a human breast.

The transmitter may transmit at a single frequency only, but preferably the transmitter is configured to transmit wave energy over a range of frequencies including the frequenc *f*.

In the preferred embodiment described below, the anti-reflection layer consists of a single layer of material. However, a multi-layer structure could also be envisaged. In this case, the total thickness of the multi-layer structure may be equal to or greater than λ/4, and one or more of the layers within the multi-layer structure may have a thickness λ/4. A multi-layer structure may give the ability to achieve better performance over a range of angles of incidence and a range of frequencies.

The method may include

a) positioning an anti-reflective layer between the object and a transmitter, typically in contact with or in close proximity to the object;
b) transmitting wave energy onto the object through the anti-reflective layer so as to generate a first reflection from a surface of the anti-reflective layer and a second reflection from a surface of the object;
c) reducing the level of the second reflection by destructive interference with the first reflection; and
d) detecting the effect of the object on the passage of the wave energy to generate an output.

If the wave energy passes through a medium between the transmitter and the anti-reflective layer having relative permittivity $\varepsilon_1$, the anti-reflective layer has a relative permittivity $\varepsilon_2$, and the surface of the object has a relative permittivity $\varepsilon_3$, then the material of the anti-reflective layer is typically chosen to have an intermediate permittivity value. That is: $\varepsilon_2$ lies between $\varepsilon_1$ and $\varepsilon_3$.

The anti-reflective layer may at least partially support the weight of the object.

A second aspect of the invention provides a method of measuring the internal structure of an object according to claim 1.

The second aspect of the invention reduces signal artefacts present in data associated with a desired point in the object, instead of acting directly on the output signals. Typically the focusing step time- or phase-aligns the output signals, and optionally the focusing step may also apply amplitude weighting factors to the output signals. Typically the additional points are selected to be in symmetrically equivalent positions in relation to the transmitters and receivers.

The data associated with the desired point may be a time varying focused signal, or a scalar quantity (such as energy) associated with the desired point. Similarly the additional data may be a time varying focused signal, or a scalar quantity associated with an additional point.

The second aspect of the invention also provides apparatus according to claim 8.

The methods of the first and second aspects of the invention may be performed in any application in which signal artefacts caused by reflections from a surface are present. For instance the object may be an area of land being surveyed to detect pipes or other buried objects. As another example the object may be part of a built structure being surveyed for faults. However typically the object is part of a human or animal body, such as a breast.

The wave energy may be ultrasound, but is more typically electromagnetic wave energy, preferably in the microwave region with a frequency higher than 1 GHz and preferably higher than 4GHz.

For a radio- or microwave-based system a relatively broadband antenna is required in order to transmit over a wide range of frequencies. In a preferred example each transmitter is a stacked slot-fed patch antenna including a first patch, a second patch, and a ground plane including a slot for coupling wave energy into the first and second patches.

Each method may be performed individually, or in combination with the other aspect of the invention.

Various embodiments of the present invention will now be described with reference to the accompanying drawings, in which:

Figure 1 is a system overview of a breast tumour imaging system;

Figure 2 is a perspective view and cross-section of a stacked-patch antenna design;

Figure 3 is a cross-sectional view illustrating similar pairs within the array;

Figure 4 is a cross-sectional view of a set of antenna elements with screens;

Figure 5 is a schematic cross-section showing a skin echo;

Figure 6 is a schematic cross-section showing an anti-reflection layer in contact with the skin; and

Figure 7 is a cross-sectional view illustrating equivalent positions in relation to the array;

## 1 Real Aperture Synthetically Organised Radar

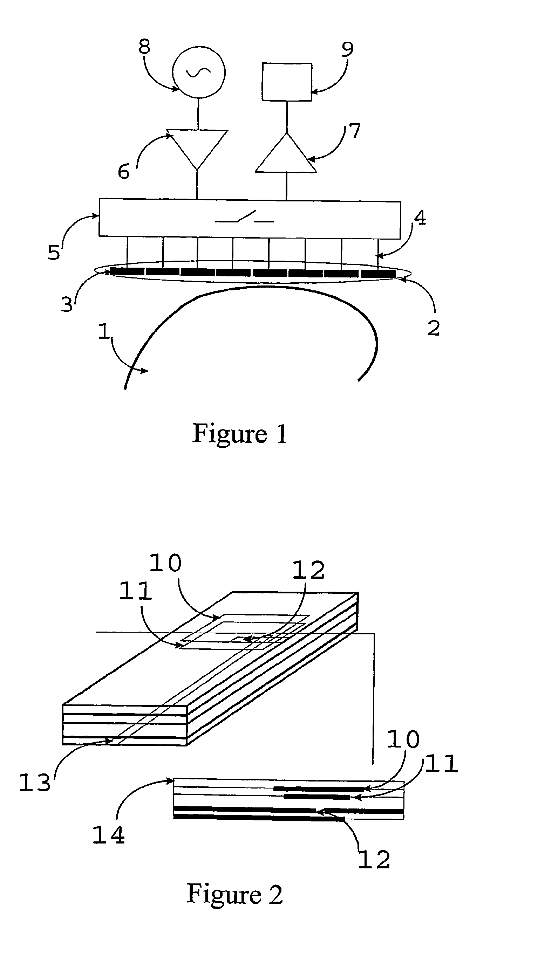

A real aperture synthetically organised radar for breast cancer detection shown in Figure 1 operates by employing an array **2** of N antennas (e.g. **3**) close to, or in contact with, the breast **1**. Each antenna in turn transmits a pulse and

the received signal $y_i(t)$ at each of the other antennas is recorded. The pulse generator **8** and the detector **9** may be time-shared, by means of a switching matrix **5** as shown in Figure 1, as may any transmit or receive path amplification (**6**, **7**).

Monostatic operation is unattractive because of the difficulty of near-simultaneous transmission and reception on the same antenna, and, since interchanging transmit and receive antennas would not produce any additional information, the total number of transmissions recorded is $N(N-1)/2$.

The recorded data is then synthetically focussed at any point of interest in the volume beneath this antenna array by time-aligning the signals $y_i(t)$, using the estimated propagation time $T_i$ from the transmit antenna to the receive antenna via any point of interest in the medium.

$$v(t) = \sum_{i=1}^{N(N-1)/2} w_i y_i(t - T_i) \qquad (1a)$$

- where $w_i$ are weighting factors that are applied to compensate for differences in the predicted attenuation between the round-trip paths between transmit and receive antennas via the point of interest, and/or to apply various optimisation criteria.

The returned signal energy associated with this point may then computed by integrating the data over a window corresponding to the transmit pulse width $\tau$:

$$V = \int_0^\tau v^2(t)\,dt \qquad (1b)$$

Alternative methods of obtaining a scalar quantity V from v(t) include computing the magnitude of a DFT at one or more frequencies or multiplying by the transmitted pulse:

$$V = \int_0^\tau v(t) \cdot x(t)\,dt \qquad (1c)$$

- where $x(t)$ is the transmitted pulse waveform.

This signal processing approach is similar in essence to other time-shift-and-sum beamforming algorithms (see for example S. C. Hagness, A. Taflove, and J. E. Bridges, Two-dimensional FDTD analysis of a pulsed microwave confocal system for breast cancer detection: fixed-focus and antenna-array sensors, IEEE Trans. on Biomed. Eng., vol. 45, pp. 1470-9, Dec. 1998; or E. C. Fear and M. A. Stuchly, Microwave detection of breast cancer, IEEE Trans. Microwave Theory and Tech., vol. 48, pp. 1854-1863, Nov. 2000). However, in utilising all possible transmit/receive combinations in the array, it differs from that described by Hagness et al and Fear et al, and the consequently increased number of observations offers additional opportunities for processing gain and clutter rejection.

**2 An Antenna Element on High-Dielectric Substrate for Radiation into the Human Breast**

The exploitation of the favourable contrast in dielectric properties between normal tissues and malignant tumour depends on radiating and receiving a sufficiently wideband waveform to achieve high resolution. This requires an antenna that radiates well into the breast over a wide band of frequencies. Conventional antennas are obviously not designed to radiate into human tissue, indeed the close proximity of human tissue usually has a detrimental effect on their operation. Additionally the antenna should be inexpensive to construct, suitable for integration into an array and low profile.

Figure 2 shows the stacked patch configuration employed for the breast imaging application. The antenna consists of two stacked patches **10**, **11** printed on a dielectric substrate of typically $\varepsilon_r = 2.2$ and separated from the ground plane by a second substrate of typically $\varepsilon_r = 9.8$, a microstrip line **13** is used to feed the lower patch via a slot **12** in the antenna ground plane. Stacked patches and slot feeds have been employed before in antenna designs, however this particular antenna was specifically designed to radiate into a medium of typically $\varepsilon_r = 10$ which approximately represents the dielectric properties of the breast tissue. A thin radome **14** of $\varepsilon_r = 9.8$ covers the antenna.

This design of the stacked patch antenna produced a bandwidth of approximately 72% and a beamwidth of approximately $\pm 35°$ in the $\phi = 0°$ plane and $\pm 30°$ in the $\phi = 90°$ plane at 7.0 GHz, calculated in tissue. Over the operating frequency range the antenna design was also found to radiate most energy, as desired, into the breast (with a front-to-back ratio better than 15 dB).

## 2 Techniques for Reducing Skin Reflections

The chief components of the signals collected at the antenna elements are mutual coupling between the antennas, reflections from skin and the tumour echo. The direct antenna couplings will not significantly interfere with the tumour echo as they occur earlier in time. The large signal artefacts caused by reflections from the skin however pose a significant challenge since they tend to mask the reflections from tumours close to skin, despite the benefits of the radar method described herein. Techniques to mitigate the skin reflections are considered in the following sections.

## 2.1.1 Skin Reflection Reduction using the Similar Paths Algorithm

An $N$ element flat array will collect $N(N-1)/2$ distinct signals arising from transmission on one antenna and reception on another. Among these paths, a number of sets of similar paths exist with approximately the same mutual coupling and skin reflections.

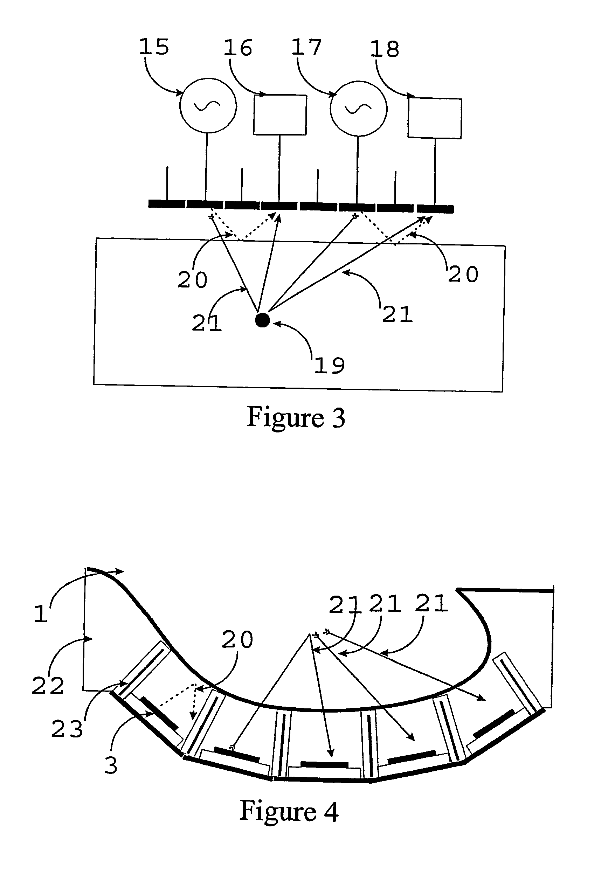

For example, any immediately adjacent pair of antennas within the array will observe similar amplitude and phase delays for the skin reflection. Similarly, as shown in Figure 3, any next-neighbour pairing of transmit and receive antennas will observe similar amplitude and phase delays for the skin reflection **20**. The contribution **21** arising from any tumour **19** will however not be the same.

While Figure 3 illustrates the principle in the simplified scenario of a linear array adjacent to a flat skin surface, the same concept may be extended to two dimensional arrays that conform to a curved surface, such as the breast (see Figure 4).

This method can be exploited to reduce the skin reflections to a considerable extent.

The signals from similar paths may be processed by either of two alternative variants, as follows:

a) minor time-shifting and alignment of the signals (to compensate for experimental tolerances) in producing a representative average signal. This average signal is then used as a calibration signal which is subtracted from each signal in the set of similar paths; or

b) for each signal in the set of similar paths, identification (by integrating the square difference between the two signals) of a second signal from within this set that most closely resembles the first. This "most similar" signal is then used as a calibration signal which is subtracted from the first; or

c) find that member of the set of similar paths with the smallest mean squared difference from all the others. This "most representative" signal is then used as a calibration signal which is subtracted from all the others.

The signals may be divided into segments in the time domain (each segment corresponding to a particular feature of the response, arising from a particular physical feature that results in coupling between the transmit and receiver antennas) and method (a) or (b) may then be applied to each segment at a time.

After the calibration signals have been subtracted, one of the focussing algorithm described in section 1 above is applied. The residual skin reflection present in the signals will be mitigated by the processing gain of the focussing algorithm.

## 2.1.2 Skin Reflection Reduction using the Equivalent Location Algorithm

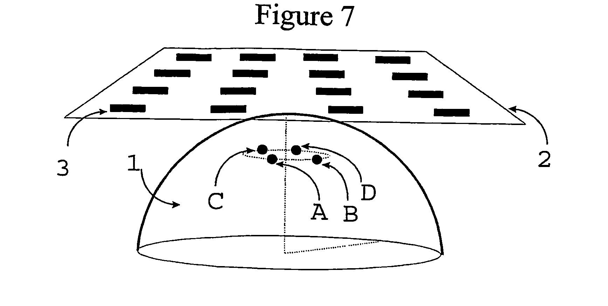

An N element array will collect $N(N-1)/2$ distinct signals arising from transmission on one antenna and reception on another. Considering a point of interest **A** within the body, as shown in Figure 7, the process of time-alignment and scaling in equation (1a) yields a focussed signal $v_1(t)$ corresponding to that point **A**.

Assume that the elements of the array are disposed around an approximately symmetrical breast in a symmetrical, or approximately-symmetrical, fashion. Then there exist one or more additional point(s) **B**, **C**, **D** with the symmetrically equivalent location to **A**, relative to the array. The focused signals $v_2(t)$, $v_3(t)$, $v_4(t)$ associated with these points would be expected to be very similar, containing, for example, the same components for the skin reflection and mutual coupling. If the elements of the array are disposed in a less symmetrical fashion, then there may be fewer than three additional points with symmetrically equivalent positions.

A calibration signal may then be generated from this subset of focused signals ($v_1(t)$, $v_2(t)$, $v_3(t)$, $v_4(t)$) and this may then be subtracted from $v_1(t)$. In this way skin reflection and mutual couplings will be much reduced.

The calibration signal may be formed, for example, by

a) minor time-shifting and alignment of the focused signals (to compensate for experimental tolerances) in producing a representative average signal. This average signal is then used as a calibration signal which is subtracted from each signal in the set; or

b) for each member of ($v_1(t)$, $v_2(t)$, $v_3(t)$, $v_4(t)$), identification (by integrating the square difference between the two signals) of a second signal from within this set that most closely resembles the first. This "most similar" signal is then used as a calibration signal which is subtracted from the first; or

c) finding that member of the set with the smallest mean squared difference from all the others. This "most representative" signal is then used as a calibration signal which is subtracted from all the others.

d) calculating a weighted average of the subset of focused signals. The contribution to any such weighted average may include only giving weight to those members of the subset which, on consideration of appropriate signal statistics, confirm that they differ little one from the other and hence are representative of the common background and of the artefacts associated therewith. This latter approach is important, for example when one of **A**, **B**, **C** or **D** corresponds to a tumour location in which case the desired tumour response is not affected since the signal is excluded from the averaging.

The calibration signal may be subtracted from $v_1(t)$ directly and the signal energy associated with this point calculated using e.g. equation (1b) or (1c). Alternatively scalar energy values may first be computed for all of ($v_1(t)$, $v_2(t)$, $v_3(t)$, $v_4(t)$), and the subtraction then performed using these scalar energy values rather than the focused signals themselves.

### 3.2 Skin Reflection Reduction using Blocking Screens

Skin reflections and mutual couplings can be considerably reduced by employing screens **23** in Figure 4 between the antenna elements. Having these screens extend to the breast **1** will eliminate or significantly reduce the skin echoes **20** but will still allow the tumour echoes **21** to reach the antenna elements. In this implementation the space **22** created between the screens, the skin and the antenna is filled with a matching liquid with similar electrical properties to healthy breast tissue, such as an emulsion of liquid paraffin and water.

The screens are thin aluminium sheets with a thin layer of radar absorbing material on both sides to reduce multiple bounces and resonance effects. Various radar absorbing materials are available, and suitable products include Emerson & Cuming ECCOSORB FGM-40 (1mm thickness), ECCOSORB BSR (0.25mm, 0.5mm thickness) and ECCOSORB FDS (0.75mm thickness). Alternative absorbing materials could be employed, including water-loaded resins.

The screens may be attached to the antenna support structure in a number of ways, such as gluing, bolting or welding.

Although the use of the screens will slightly reduce the half power beamwidths of the antenna elements and hence reduce the number of antenna pairs associated with any given location, the benefits are still significant.

Computer simulations of the antenna elements and associated set of screens show that a well-behaved and almost frequency-independent radiation pattern is obtained over the operating frequency range from 4.5GHz to 9.5GHz.

Numerical simulations were conducted to analyse the merits of screens, employing a FDTD model developed for the analysis of breast imaging. The computer simulations and FDTD model are described in detail in R. Nilavalan, J. Leendertz, I. J. Craddock, A. Preece, R. Benjamin Numerical Analysis of Microwave Detection of Breast Tumours Using Synthetic Focussing Techniques , Proceedings of the IEEE AP-S International Symposium and USNCIURSI National Radio Science Meeting, Monterey, California, USA, June 2004.

**Table 1 Tumour to skin power ratio**

| Tumour Size Diameter (mm) | Without screens (dB) | With screens (dB) |
| --- | --- | --- |
| 2 | -47.1 | -27.0 |
| 3 | -40.8 | -19.6 |
| 4 | -32.4 | -10.9 |
| 5 | -29.5 | -8.7 |

The calculated tumour to skin power ratios are given in Table 1 (the signal from the tumour can be calculated exactly using a background subtraction technique) - these can be seen to yield a 20dB reduction in the power of the skin reflection relative to the signal from the tumour.

**3.3 Skin Reflection Reduction using an Anti-Reflection Layer**

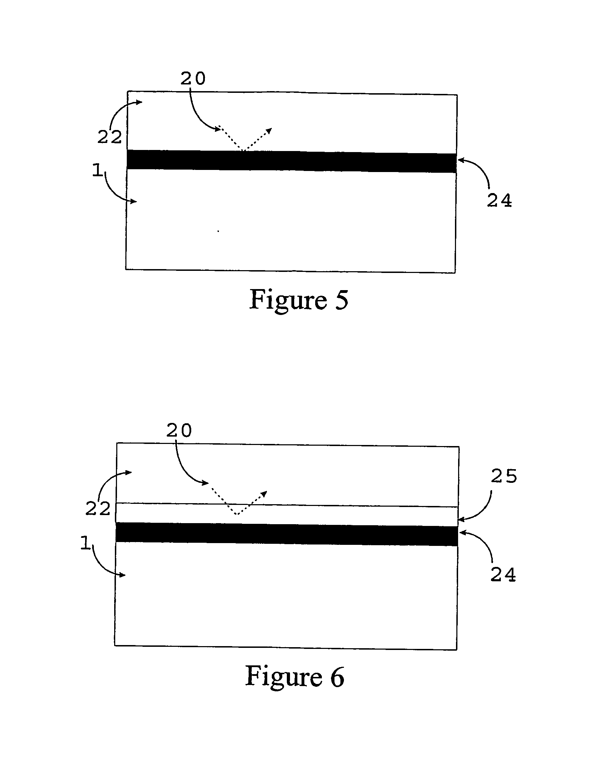

As shown in simplified form by Figure 5, skin echoes **20** arise from the three layered structure comprising the matching liquid **22** (with assumed relative permittivity $\varepsilon_1$), skin **24** (with assumed relative permittivity $\varepsilon_3$) and the breast tissue **1**. The total echo comprises reflections from the two interfaces and the multiple bounces between these interfaces.
Since the skin is an attenuating medium, the largest echo is a result of the single reflection from the upper face of the skin. This echo may be reduced by introducing an Anti-Reflection (AR) layer **25** next to the skin, as shown in Figure 6.
By considering just this largest echo, approximate theoretical analysis shows that a minimum reflection is achieved when the relative permittivity $\varepsilon_2$ of the AR layer is $\varepsilon_2 = \sqrt{\varepsilon_1 \varepsilon_3}$ and the thickness d of the layer satisfies:

$$e^{-4\pi jd\sqrt{\varepsilon_2}/\lambda} = -1$$

- where $\lambda$ is the free-space wavelength.
This approximate result implies, for lossless media, that $d$ should be a quarter-wavelength in the AR layer and to a reasonable approximation this holds for lossy media as well. More rigorous analysis of the reflection and transmission mechanisms will lead to slightly different choices for $d$ and $\varepsilon_2$.
To validate this approach an experiment was devised using a water- and aluminium-loaded resin-based material for the AR layer. By adjusting the water and aluminium content this layer can be made with parameters that were a reasonable approximation to the desired values. The thickness of the layer was of the order of 3mm (approximately $\lambda/4$ at the mid-point frequency of 6GHz).
The reflectivities of the skin phantom with the antireflection layer, and of a layer of skin phantom alone, were measured in a bath of breast tissue phantom medium using a network analyser.
Although the properties of the layer were optimised for a single frequency of 6GHz, the antireflection layer yielded a reduction of over 10dB in the reflected signal from the skin across the frequency range 4.5GHz to 7GHz. Even outside of this frequency range the performance was generally better with the AR layer present.
In practice the patient is envisaged as lying in a prone position and for comfort as well as experimental precision, it is envisaged that the breast will be supported by a gently curved shell, probably created from a rigid moulded resin material. It is apparent from the above results that a shell with antireflection properties would be a particularly appropriate choice.

**Claims**

**1.** A method of measuring the internal structure of an object (1), the method including the steps of:

a) energising a transmitter (3) so as to transmit wave energy onto the object;
b) detecting the effect of the object on the passage of the wave energy with a plurality of receivers (3) to generate a plurality of output signals;
c) focusing the output signals to generate data associated with a desired point (A) in the object;
d) selecting one or more additional points (B, C, D) in the object;
e) focusing the output signals to generate additional data each associated with a respective additional point; and
f) reducing signal artifacts by

i) generating calibration data from the additional data, and
ii) subtracting the calibration data from the data associated with the desired point,

wherein each additional point has a symmetrically equivalent position, in relation to the transmitter and receivers, to the desired point so that the data associated with these points would be expected to be very similar.

**2.** A method according to claim 1 wherein step i) includes selecting data associated with one of the additional points (B, C, D).

**3.** A method according to claim 1 wherein step i) includes calculating an average of the additional data.

**4.** A method according to claim 3 wherein the average is a weighted average.

**5.** A method according to any preceding claim wherein the wave energy contains frequency components spanning a range of frequencies.

**6.** A method according to claim 5 wherein the wave energy contains frequency components spanning a range having a width which is greater than 50% of the centre-frequency.

**7.** A method according to claim 5 or 6 wherein the wave energy contains frequency components spanning a range having a width which is greater than 1 GHz, and preferably greater than 4 GHz.

**8.** Apparatus for measuring the internal structure of an object (1), the apparatus including:

a) a transmitter (3) configured to transmit wave energy onto the object,
b) a plurality of receivers (3) configured to detect the effect of the object on the passage of the wave energy and generate a plurality of output signals; and
c) a processor configured to:

i) focus the output signals to generate data associated with a desired point (A) in the object;
ii) select one or more additional points (B, C, D) in the object;
iii) focus the output signals to generate additional data associated with each additional point; and
iv) reduce signal artifacts by

(1) generating calibration data from the additional data, and
(2) subtracting the calibration data from the data associated with the desired point,

wherein each additional point has a symmetrically equivalent position, in relation to the transmitter and receivers, to the desired point so that the data associated with these points would be expected to be very similar.

**9.** A method of measuring the internal structure of an object (1), the method including the steps of:

a) energising a transmitter (3) so as to transmit wave energy onto the object, the wave energy containing frequency components spanning a range of frequencies;
b) detecting the effect of the object on the passage of the wave energy with a plurality of receivers (3) to generate a plurality of output signals; and
c) reducing signal artifacts by

i) selecting a subset of output signals, each output signal in the subset corresponding with a receiver which is spaced apart from the transmitter by a similar distance, wherein the number of output signals in the subset is smaller than the total number of output signals generated by the receivers,
ii) generating one or more calibration signal from the subset of output signals, the calibration signal(s) containing frequency components spanning a range of frequencies, and
iii) subtracting the calibration signal(s) from one or more of the output signals in the subset.

**10.** A method according to claim 9 wherein step ii) includes selecting one of the output signals in the subset.

**11.** A method according to claim 9 wherein step ii) includes calculating an average of the subset of output signals.

**12.** A method according to claim 11 wherein the average is a weighted average.

**13.** A method according to any of claims 9 to 12 wherein the calibration signal contains frequency components spanning a range having a width which is greater than 50% of the centre-frequency.

**14.** A method according to any of claims 9 to 13 wherein the calibration signal contains frequency components spanning a range having a width which is greater than 1 GHz, and preferably greater than 4 GHz.

**15.** Apparatus for measuring the internal structure of an object (1), the apparatus including:

a) a transmitter (3) configured to transmit wave energy onto the object, the wave energy containing frequency components spanning a range of frequencies;

b) a plurality of receivers (3) configured to detect the effect of the object on the passage of the wave energy and generate a plurality of output signals; and

c) a processor configured to reduce signal artifacts by:

i) selecting a subset of output signals, each output signal in the subset corresponding with a receiver which is spaced apart from the transmitter by a similar distance, wherein the number of output signals in the subset is smaller than the total number of output signals generated by the receivers,

ii) generating one or more calibration signals from the subset of output signals, the calibration signal(s) containing frequency components spanning a range of frequencies, and

iii) subtracting the calibration signal(s) from one or more of the output signals in the subset.

**16.** A method according to any of the preceding method claims wherein the object is part of a human or animal body.

**17.** A method according to claim 16 wherein the object (1) is a breast.

**Patentansprüche**

**1.** Verfahren zum Messen der internen Struktur eines Objekts (1), wobei das Verfahren die Schritte aufweist:

a) Aktivieren eines Senders (3), um so Wellenenergie auf das Objekt zu richten,

b) Detektieren des Effekts des Objekts auf den Durchgang der Wellenenergie mit einer Mehrzahl von Empfängern (3), um eine Mehrzahl von Ausgabesignalen zu erzeugen,

c) Fokussieren der Ausgabesignale, um Daten, die einem gewünschten Punkt (A) in dem Objekt zugeordnet sind, zu erzeugen,

d) Auswählen von einem oder mehreren zusätzlichen Punkten (B, C, D) in dem Objekt,

e) Fokussieren der Ausgabesignale, um zusätzliche Daten zu erzeugen, die jeweils einem der zusätzlichen Punkte zugeordnet sind, und

f) Reduzieren von Signalartefakten, indem

i) aus den zusätzlichen Daten Kalibrationsdaten erzeugt werden und

ii) die Kalibrationsdaten von den dem gewünschten Punkt zugeordneten Daten subtrahiert werden,

wobei jeder zusätzliche Punkt eine, in Bezug auf den Sender und die Empfänger, symmetrisch äquivalente Positionzu dem gewünschten Punkt hat, so dass die diesen Punkten zugeordneten Daten als sehr ähnlich zu erwarten wären.

**2.** Verfahren nach Anspruch 1, wobei Schritt i) beinhaltet, Daten, die einem der zusätzlichen Punkte (B, C, D) zugeordnet sind, zu selektieren.

**3.** Verfahren nach Anspruch 1, wobei Schritt i) beinhaltet, einen Mittelwert der zusätzlichen Daten zu berechnen.

**4.** Verfahren nach Anspruch 3, wobei der Mittelwert ein gewichteter Mittelwert ist.

**5.** Verfahren nach einem der vorhergehenden Ansprüche, wobei die Wellenenergie Frequenzkomponenten enthält, die einen Bereich von Frequenzen überspannen.

**6.** Verfahren nach Anspruch 5, wobei die Wellenenergie Frequenzkomponenten enthält, die einen Bereich mit einer Breite, die größer als 50% der Mittenfrequenz ist, überspannen.

**7.** Verfahren nach Anspruch 5 oder 6, wobei die Wellenenergie Frequenzkomponenten enthält, die einen Bereich mit einer Breite, die größer als 1 GHz und vorzugsweise größer als 4 GHz ist, überspannen.

**8.** Vorrichtung zum Messen der inneren Struktur eines Objekts (1), wobei die Vorrichtung aufweist:

a) einen Sender (3), der dazu ausgestaltet ist, um Wellenenergie auf ein Objekt zu richten,

b) eine Mehrzahl von Empfängern (3), die dazu ausgestaltet sind, um den Effekt des Objekts auf den Durchgang der Wellenenergie zu detektieren und eine Mehrzahl von Ausgabesignalen zu erzeugen, und

c) einen Prozessor, der dazu eingerichtet ist, um:

i) die Ausgabesignale zu fokussieren, um Daten zu erzeugen, die einem gewünschten Punkt (A) in dem Objekt zugeordnet sind,

ii) einen oder mehrere zusätzliche Punkte (B, C, D) in dem Objekt zu selektieren,

iii) die Ausgabesignale zu fokussieren, um zusätzliche Daten zu erzeugen, die jedem zusätzlichen Punkt zugeordnet sind, und

iv) Signalartefakte zu reduzieren, indem

(1) Kalibrationsdaten aus den zusätzlichen Daten erzeuget werden und

(2) die Kalibrationsdaten von den dem gewünschten Punkt zugeordneten Daten subtrahiert werden,

wobei jeder zusätzliche Punkt, in Bezug auf den Sender und die Empfänger, eine symmetrisch äquivalente Position zu dem gewünschten Punkt hat, so dass die diesen Punkten zugeordneten Daten als sehr ähnlich zu erwarten wären.

9. Verfahren zum Messen der inneren Struktur eines Objekts (1), wobei das Verfahren die Schritte aufweist:

a) Aktivieren eines Senders (3), um so Wellenenergie auf das Objekt zu richten, wobei die Wellenenergie Frequenzkomponenten enthält, die einen Bereich von Frequenzen überspannen,

b) Detektieren des Effekts des Objekts auf den Durchgang der Wellenenergie mit einer Mehrzahl von Empfängern (3), um eine Mehrzahl von Ausgabesignalen zu erzeugen, und

c) Reduzieren von Signalartefakten, indem

i) eine Untermenge von Ausgabesignalen selektiert wird, wobei jedes Ausgabesignal in der Untermenge einem Empfänger entspricht, der mit einem ähnlichen Abstand von dem Sender entfernt ist, wobei die Anzahl von Ausgabesignalen in der Untermenge kleiner als die Anzahl von von den Empfängern erzeugten Ausgabesignalen ist,

ii) ein oder mehrere Kalibrationssignale aus der Untermenge der Ausgabesignale erzeugt werden, wobei das (die) Kalibrationssignal(e) Frequenzkomponenten enthält (enthalten), die einen Bereich von Frequenzen überspannen, und

iii) das(die) Kalibrationssignal(e) von einem oder mehreren der Ausgabesignale in der Untermenge subtrahiert werden.

10. Verfahren nach Anspruch 9, wobei Schritt ii) beinhaltet, eines der Ausgabesignale in der Untermenge auszuwählen.

11. Verfahren nach Anspruch 9, wobei Schritt ii) beinhaltet, einen Mittelwert der Untermenge der Ausgabesignale zu berechnen.

12. Verfahren nach Anspruch 11, wobei der Mittelwert ein gewichteter Mittelwert ist.

13. Verfahren nach einem der Ansprüche 9 bis 12, wobei das Kalibrationssignal Frequenzkomponenten enthält, die einen Bereich mit einer Breite überspannen, die größer als 50% der Mittenfrequenz ist.

14. Verfahren nach einem der Ansprüche 9 bis 13, wobei das Kalibrationssignal Frequenzkomponenten enthält, die einen Bereich mit einer Breite überspannen, die größer als 1 GHz, und vorzugsweise größer als 4 GHz ist.

15. Vorrichtung zum Messen der internen Struktur eines Objekts (1), wobei die Vorrichtung aufweist:

a) einen Sender (3), der dazu ausgestaltet ist, um Wellenenergie auf das Objekt zu richten, wobei die Wellenenergie Frequenzkomponenten enthält, die einen Bereich von Frequenzen überspannen,

b) eine Mehrzahl von Empfängern (3), die dazu ausgestaltet sind, um den Effekt des Objekts auf den Durchgang der Wellenenergie zu detektieren und eine Mehrzahl von Ausgabesignalen zu erzeugen, und

c) einen Prozessor, der dazu eingerichtet ist, Signalartefakte zu reduzieren, indem:

i) eine Untermenge von Ausgabesignalen selektiert wird, wobei jedes Ausgabesignal in der Untermenge einem Empfänger entspricht, der mit einem ähnlichen Abstand von dem Sender entfernt ist, wobei die Anzahl der Ausgabesignale in der Untermenge kleiner als die Gesamtzahl der Ausgabesignale ist, die von den Empfängern erzeugt werden,

ii) ein oder mehrere Kalibrationssignale aus der Untermenge von Ausgabesignalen erzeugt werden, wobei das (die) Kalibrationssignal(e) Frequenzkomponenten enthält (enthalten), die einen Bereich von Frequenzen überspannen, und

iii) das (die) Kalibrationssignal(e) von einem oder mehreren der Ausgabesignale in der Untermenge abgezogen wird (werden).

16. Verfahren nach einem der vorhergehenden Verfahrensansprüche, wobei das Objekt Teil eines menschlichen oder tierischen Körpers ist.

17. Verfahren nach Anspruch 16, wobei das Objekt (1) eine Brust ist.


**Revendications**

1. Procédé de mesure de la structure interne d'un objet (1), le procédé incluant les étapes de :

a) fournir de l'énergie à un transmetteur (3) de manière à transmettre de l'énergie ondulatoire sur l'objet ;
b) détecter l'effet de l'objet sur le passage de l'énergie ondulatoire avec une pluralité de récepteurs (3) pour générer une pluralité de signaux de sortie ;
c) focaliser les signaux de sortie pour générer des données associées à un point souhaité (A) dans l'objet ;
d) sélectionner un ou plusieurs points supplémentaires (B, C, D) dans l'objet ;
e) focaliser les signaux de sortie pour générer des données supplémentaires associées chacune à un point supplémentaire respectif ; et
f) réduire des artefacts de signal en

i) générant des données de calibrage à partir des données supplémentaires, et
ii) soustrayant les données de calibrage des données associées au point souhaité,

dans lequel chaque point supplémentaire a une position symétriquement équivalente, par rapport au transmetteur et aux récepteurs, au point souhaité de sorte que l'on s'attendrait à ce que les données associées à ces points soient très similaires.

2. Procédé selon la revendication 1, dans lequel l'étape i) inclut la sélection de données associées à l'un des points supplémentaires (B, C, D).

3. Procédé selon la revendication 1, dans lequel l'étape i) inclut le calcul d'une moyenne des données supplémentaires.

4. Procédé selon la revendication 3, dans lequel la moyenne est une moyenne pondérée.

5. Procédé selon l'une quelconque des revendications précédentes, dans lequel l'énergie ondulatoire contient des composantes fréquentielles couvrant une plage de fréquences.

6. Procédé selon la revendication 5, dans lequel l'énergie ondulatoire contient des composantes fréquentielles couvrant une plage ayant une largeur qui est supérieure à 50 % de la fréquence centrale.

7. Procédé selon la revendication 5 ou 6, dans lequel l'énergie ondulatoire contient des composantes fréquentielles couvrant une plage ayant une largeur qui est supérieure à 1 GHz, et de préférence supérieure à 4 GHz.

8. Appareil pour mesurer la structure interne d'un objet (1), l'appareil incluant :

a) un transmetteur (3) configuré pour transmettre de l'énergie ondulatoire sur l'objet,
b) une pluralité de récepteurs (3) configurés pour détecter l'effet de l'objet sur le passage de l'énergie ondulatoire et générer une pluralité de signaux de sortie ; et
c) un processeur configuré pour :

i) focaliser les signaux de sortie pour générer des données associées à un point souhaité (A) dans l'objet ;

ii) sélectionner un ou plusieurs points supplémentaires (B, C, D) dans l'objet ;

iii) focaliser les signaux de sortie pour générer des données supplémentaires associées à chaque point supplémentaire ; et

iv) réduire des artefacts de signal en

(1) générant des données de calibrage à partir des données supplémentaires, et

(2) soustrayant les données de calibrage des données associées au point souhaité,

dans lequel chaque point supplémentaire a une position symétriquement équivalente, par rapport à l'émetteur et aux récepteurs, au point souhaité de sorte que l'on s'attendrait à ce que les données associées à ces points soient très similaires.

9. Procédé de mesure de la structure interne d'un objet (1), le procédé incluant les étapes de :

a) fournir de l'énergie à un transmetteur (3) de manière à transmettre de l'énergie ondulatoire sur l'objet, l'énergie ondulatoire contenant des composantes fréquentielles couvrant une plage de fréquences ;

b) détecter l'effet de l'objet sur le passage de l'énergie ondulatoire avec une pluralité de récepteurs (3) pour générer une pluralité de signaux de sortie ; et

c) réduire des artefacts de signal en

i) sélectionnant un sous-ensemble de signaux de sortie, chaque signal de sortie dans le sous-ensemble correspondant à un récepteur qui est espacé du transmetteur d'une distance similaire, dans lequel le nombre de signaux de sortie dans le sous-ensemble est plus petit que le nombre total de signaux de sortie générés par les récepteurs,

ii) générant un ou plusieurs signaux de calibrage à partir du sous-ensemble de signaux de sortie, le(s) signal (signaux) de calibrage contenant des composantes fréquentielles couvrant une plage de fréquences, et

iii) soustrayant le(s) signal (signaux) de calibrage d'un ou plusieurs des signaux de sortie dans le sous-ensemble.

10. Procédé selon la revendication 9, dans lequel l'étape ii) inclut la sélection de l'un des signaux de sortie dans le sous-ensemble.

11. Procédé selon la revendication 9, dans lequel l'étape ii) inclut le calcul d'une moyenne du sous-ensemble de signaux de sortie.

12. Procédé selon la revendication 11, dans lequel la moyenne est une moyenne pondérée.

13. Procédé selon l'une quelconque des revendications 9 à 12, dans lequel le signal de calibrage contient des composantes fréquentielles couvrant une plage ayant une largeur qui est supérieure à 50 % de la fréquence centrale.

14. Procédé selon l'une quelconque des revendications 9 à 13, dans lequel le signal de calibrage contient des composantes fréquentielles couvrant une plage ayant une largeur qui est supérieure à 1 GHz, et de préférence supérieure à 4 GHz.

15. Appareil pour mesurer la structure interne d'un objet (1), l'appareil incluant :

a) un transmetteur (3) configuré pour transmettre de l'énergie ondulatoire sur l'objet, l'énergie ondulatoire contenant des composantes fréquentielles couvrant une plage de fréquences ;

b) une pluralité de récepteurs (3) configurés pour détecter l'effet de l'objet sur le passage de l'énergie ondulatoire et générer une pluralité de signaux de sortie ; et

c) un processeur configuré pour réduire des artefacts de signal en :

i) sélectionnant un sous-ensemble de signaux de sortie, chaque signal de sortie dans le sous-ensemble correspondant à un récepteur qui est espacé de l'émetteur d'une distance similaire, dans lequel le nombre de signaux de sortie dans le sous-ensemble est plus petit que le nombre total de signaux de sortie générés par les récepteurs,

ii) générant un ou plusieurs signaux de calibrage à partir du sous-ensemble de signaux de sortie, le(s) signal (signaux) de calibrage contenant des composantes fréquentielles couvrant une plage de fréquences, et

iii) soustrayant le(s) signal (signaux) de calibrage d'un ou plusieurs des signaux de sortie dans le sous-ensemble.

16. Procédé selon l'une quelconque des revendications précédentes, dans lequel l'objet est une partie d'un corps humain ou animal.

17. Procédé selon la revendication 16, dans lequel l'objet (1) est un sein.

Figure 1

Figure 2

Figure 3

Figure 4

Figure 5

Figure 6

Figure 7

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 5920285 A
- WO 03009753 A

### Non-patent literature cited in the description

- **M. BROWN ; F. HOUN ; E. SICKLES ; L. KESSLER.** Screening mammography in community practice. *Amer. J. Roentgen,* December 1995, vol. 165, 1373-1377
- **P. T. HUYNH ; A. M. JAROLIMEK ; S .DAYE.** The false negative mammogram. *Radiograph,* 1998, vol. 18, 1137-1154
- **E. BANKS et al.** Influence of personal characteristics of individual women on sensitivity and specificity of mammography in the Million Women Study: cohort study. *British Medical Journal,* August 2004, vol. 329 (7464), 477
- **XU LI ; S. C. HAGNESS.** A confocal microwave imaging algorithm for breast cancer detection. *IEEE Microwave & Wireless Components Lett.,* March 2001, vol. 11, 130-2
- **E. C. FEAR ; M. A. STUCHLY.** Microwave system for breast tumour detection. *IEEE Microwave & Guided Wave Lett.,* November 1999, vol. 9, 470-2
- **P. M. MEANEY ; M. W. FANNING ; D. LI ; S. P. POPLACK ; K. D. PAULSEN.** Clinical prototype for active microwave imaging of the breast. *IEEE Trans. on Microwave Theory and Tech.,* November 2000, vol. 48, 1841-1853

- **S. C. HAGNESS ; A. TAFLOVE ; J. E. BRIDGES.** Two-dimensional FDTD analysis of a pulsed microwave confocal system for breast cancer detection: fixed-focus and antenna-array sensors. *IEEE Trans. on Biomed. Eng.,* December 1998, vol. 45, 1470-9
- **R. BENJAMIN ; I. J. CRADDOCK ; G. S. HILTON ; S. LITOBARSKI ; E. MCCUTCHEON ; R. NILAVALAN ; G. N. CRISP.** Microwave detection of buried mines using non-contact, synthetic near-field focusing. *IEE Proceedings: Radar, Sonar & Navigation,* August 2001, vol. 148, 233-40
- **E. C. FEAR ; M. A. STUCHLY.** Microwave detection of breast cancer. *IEEE Trans. Microwave Theory and Tech.,* November 2000, vol. 48, 1854-1863
- **R. NILAVALAN ; J. LEENDERTZ ; I. J. CRADDOCK ; A. PREECE ; R. BENJAMIN.** Numerical Analysis of Microwave Detection of Breast Tumours Using Synthetic Focussing Techniques. *Proceedings of the IEEE AP-S International Symposium and USNCIURSI National Radio Science Meeting,* June 2004